(19) Europäisches Patentamt
European Patent Office
Office européen des brevets

(11) Publication number: **0 319 136**
**A1**

(12) # EUROPEAN PATENT APPLICATION

(21) Application number: **88310078.6**

(51) Int. Cl.4: **A61K 9/50**

(22) Date of filing: **26.10.88**

(30) Priority: **28.10.87 FI 874742**

(43) Date of publication of application:
**07.06.89 Bulletin 89/23**

(84) Designated Contracting States:
**AT BE CH DE ES FR GB GR IT LI LU NL SE**

(71) Applicant: **K & V LICENCING OY**
**P.O. Box 34**
**SF-02660 Espoo(FI)**

(72) Inventor: **Virtanen, Jorma**
**Ristiaallokonkatu 4 C 71**
**SF-02320 Espoo(FI)**
Inventor: **Kinnunen, Paavo**
**Punarinnantie 4**
**SF-02660 Espoo(FI)**

(74) Representative: **Crisp, David Norman et al**
**D. YOUNG & CO. 10 Staple Inn**
**London, WC1V 7RD(GB)**

(54) **Novel liposomes and a process for their preparation.**

(57) Phosphatidyl glycerol-containing liposomes wherein their wall structure comprises a specific isomer, namely the sn-3-phosphatidyl-sn-1′-glycerol-isomer. This type of liposome exhibits superior stability and has a lesser tendency to bind sodium ions.

EP 0 319 136 A1

## Novel liposomes and a process for their preparation

The present invention concerns novel phosphatidyl-containing liposomes and a process for their preparation.

Amphiphilic or amphipathic compounds, such as for example phospholipids, comprise two physicochemically differing parts, viz. a hydrophobic, i.e. a water repelling part, and a hydrophilic, i.e. a water soluble part. As an example of a hydrophobic part, alkyl chains of fatty acids of phosphatidyl glycerol may be mentioned, and as a hydrophilic part the phosphoglycerol moiety thereof. When dispersing a suitable amphiphilic compound in water, microscopic vesicles, liposomes, are formed spontaneously which have diameters of about 100 to 10,000 Å. The wall of a liposome comprises a bilayer membrane or film formed by the amphiphilic compound. In this bilayer membrane the polar hydrophilic heads of the amphiphilic compound extend outwardly, i.e. they are in contact with the aqueous phase enclosed by and surrounding the liposomes, whereas the hydrophobic parts extend inwardly into the wall structure.

Liposomes are divided by their type into different subclasses: one such subclass is formed by unilamellar liposomes comprising only a single lipid bilayer, and a second subclass is formed by multilamellar liposomes comprising several lipid bilayers. The unilamellar liposomes are furthermore divided into small unilamellar liposomes or vesicles, SUV, and large unilamellar liposomes or vesicles, LUV.

Common for the different liposome types is the fact that the surrounding liposome membrane structure is poorly penetrated by water soluble compounds. For pharmaceutical use, most important are SUV and LUV liposomes. There are several advantages associated with the use of liposomes for encapsulation of pharmaceuticals for administration. Thus, liposomes facilitate the penetration of an encapsulated active substance into a cell. By using liposomes, therapeutic treatment may be locally targeted to the site of illness and thus toxic reactions in other parts of the body may be avoided. Also retarded release of the active substance may be achieved and an effective, but non-toxic systemic level may be reached and thus medical intoxication avoided. By means of liposomes, sensitive drugs may often be protected against deactivation.

Various methods have been developed for the preparation of liposomes and for obtaining a homogenous size distribution thereof. Typically liposomes may be prepared by dispersing phospholipid(s) to be used as the wall material in an aqueous medium which optionally contains a substance to be encapsulated, which medium is, or is buffered to, a pH-value close to neutral. Dispersions may be effected sonically.

Most attention in the preparation of liposomes for pharmaceutical use has hitherto been directed to the use of phospholipids as amphiphilic compounds. Phospholipids are compounds which are natural to an organism, and thus they do not generally give rise to foreign matter reactions. Typical such phospholipids are phosphatidic acid, lecithin (phosphatidyl choline) and cephalin (phosphatidyl serine). Also phosphatidyl glycerols are often used for the preparation of liposomes. Important from the viewpoint of liposomal stability is the fact that an acidic phospholipid forms part of the membrane so that the liposome formed will then carry a negative net charge on its surface. As a consequence, the liposomes will tend to repel each other. This in turn prevents, on the one hand, aggregation of the liposome vesicles (flocculation), and on the other hand their fusion, i.e. the melting together of the membrane structures.

A typical phospholipid composition for the preparation of liposomes may contain, for example, lecithin, phosphatidyl glycerol and cholesterol, for example with a molar ratio (%) of 60:10:30. Phosphatidyl glycerol commonly used for the preparation of liposomes has, besides at the sn-2-carbon atom of the glycerol structure, also an L, D-configuration in the polar end glycerol moiety. Phosphatidyl glycerol is conventionally prepared from lecithin by so called trans-phosphatidylation using phospholipase D-enzyme. As a result of the trans-phospatidylation reaction, however, a product is formed which is racemic with respect to the polar end glycerol moiety: the phosphatidyl glycerol mixture obtained for use in the preparation of liposomes namely contains both the 1′- and the 3′-form, as well as the 2′-form to some degree. During the trans-phosphatidylation also some cardiolipin is formed, which is an antigenic compound and the presence of which in liposomes may be quite detrimental.

We have now surprisingly discovered that by using the 1′-isomer of phosphatidyl glycerol for the preparation of liposomes some remarkable advantages are obtained. We have particularly discovered that the 1′- and 3′-isomers differ from each other substantially in their physicochemical properties. A liposome made from the 3′-isomer binds, due to its stereochemical structure, $Na^+$-ions to its surface much more tightly than the 1′-isomer, thus neutralizing the negative charge on the liposome surface. As a consequence of this a liposomal

dispersion containing the 3′-isomer is substantially more crystalline in the presence of $Na^+$-ions than a corresponding dispersion made from the 1′-isomer, thus leading to the formation of undesirable agglomeration and the fusion of liposomes in a dispersion. It has thus been shown that a liposome dispersion made from the 3′-isomer of a typical phosphatidyl glycerol has a storage stability of only a few days, that of a 1:1-mixture of the 1′- and 3′-isomer of a few weeks, and finally that of the plain 1′-isomer of several months.

$Na^+$-ions are common in all organisms. They are the major cations in extracellular fluids and play a major role in affecting ionic and osmotic balances. Thus if the 3'-isomer of phosphatidyl glycerol, which binds tightly sodium ions, is administered as a liposomal carrier for e.g. pharmaceuticals, this may affect detrimentally the ionic balance of an organism. On the other hand, by using according to the invention, liposomes containing the 1′-isomer of phosphatidyl glycerol as a carrier for pharmaceuticals, the transfer of $Na^+$-ion binding lipids into the organism may be avoided.

Further, only the 1′-isomer exists in nature, wherefore the 3′-isomer cannot be considered a natural substance to which the biological systems would automatically adapt themselves. Even if the liposomes were to contain a mixture, in which the phosphatidyl glycerol was formed to a major part by the 1′-isomer, and only to a minor part by the 3′-isomer, it is conceivable that the 3′-isomer, due to its slower metabolism, might accumulate in the organism, which may prove detrimental.

Thus the invention concerns in its first aspect a liposome, the wall material of which consists of a specific isomer of phosphatidyl glycerol, namely the sn-3-phosphatidyl-sn-1′-glycerol isomer. In its second aspect the invention concerns a process for the preparation of the novel liposome, which process is characterized in that an sn-3-phosphatidyl-sn-1′-glycerol-isomer is dispersed in-an aqueous medium, and liposomes thus formed are collected.

The sn-3-phosphatidyl-sn-1′-glycerol used according to the invention may be characterized by the general formula

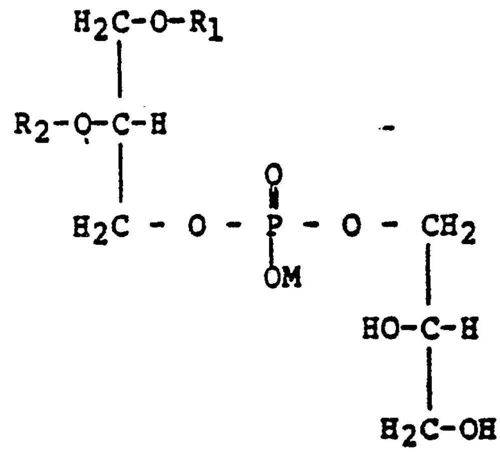

wherein $R_1$ and $R_2$ are independently straight chained alkyl-, alkenyl-, alkanoyl- or alkenoyl groups comprising about 4 to 24 carbon atoms, whereby $R_1$ and $R_2$ together contain at least 16 carbon atoms; and M is H, $NH_4^+$, $Na^+$, $K^+$, $Ca^{++}$ or $Mg^{++}$.

Preferably $R_1$ and $R_2$ are similar and contain from 12 to 18 carbon atoms in a straight chain containing from 0 to 3 double bonds, and more preferably they are residues of tetradecane (i.e. myristic acid), hexadecane (i.e. palmitic acid), or octadecene (i.e. oleic acid).

Thus, preferred compounds for use according to the invention include:

1,2-dimyristoyl-sn-glycero-3-phosphatidyl-sn-1′-glycerol (1′-DMPG),
1,2-dioleoyl-sn-glycero-3-phosphatidyl-sn-1′-glycerol (1′-DOPG),
1,2-dipalmitoyl-sn-glycero-3-phosphatidyl-sn-1′-glycerol (1′-DPPG),
1-palmitoyl-2-oleoyl-sn-glycero-3-phosphatidyl-sn-1′-glycerol (1′-POPG), and mixtures thereof.

The sn-3-phosphatidyl-sn-1′-glycerols to be used according to the invention are commercially available (e.g. from KSV-Chemicals Oy, Helsinki, Finland).

According to the invention, the liposomes may be prepared e.g. by dissolving a sn-3-phosphatidyl-sn-1′-glycerol in a suitable organic solvent in which the compound is soluble, such as in a halogenated aliphatic, cycloaliphatic or aromatic solvent, an alcohol, ester or ether, for example in chloroform or dichloromethane. Thereafter the organic solvent is removed by evaporation, for example in a nitrogen stream or in vacuo. The product obtained is thereafter dispersed in an aqueous phase, e.g. at a temperature between room temperature and about 60 °C, conveniently about 30 °C, which aqueous phase preferably has been buffered or is buffered with a buffer to a pH-value close to neutral, i.e. to about 7.0.

The formation of liposomes takes place spontaneously or its formation may be facilitated by shaking or stirring, for example sonically. The formation of liposomes may also be achieved by using other methods known in the art for preparing liposomes, such as high pressure filtration, reversed phase evaporation or detergent dialysis. In the process some multilamellar liposomes are simultaneously formed and they may be removed for example by centrifuging to obtain a uniformly unilamellar liposome dispersion. The size of unilamellar liposomes may be varied e.g. by varying the concentration of the phosphatidyl-1′-glycerol and thus a dispersion may be prepared containing either a high degree of SUV- or of LUV-liposomes depending on the intended use. The SUV-liposomes may also be separated from the LUV-liposomes using processes known in the art, for example by gel filtration or by ultracentrifugation. A general review as regards the preparation of liposomes, their size distribution characterization and the obtaining of a defined size distribution, may be found in Research Monographs in Cell and Tissue Physiology, Volume 7: Liposomes: From physical structure to therapeutic applications, editor C. G. Knight, 1981, Elsevier/North Holland Biomedical Press.

The liposomes prepared according to the invention may be convenient carrier systems for encapsulating various lipophilic and water soluble substances in a known manner. For this purpose the substance to be encapsulated may be mixed with the phosphatidyl-1′-glycerol and the mixture obtained dispersed in an aqueous phase, or the phosphatidyl-1′-glycerol may be dispersed in an aqueous phase containing the substance to be encapsulated.

In the liposomes according to the invention various active agents may be encapsulated, for example dyes, pharmaceuticals, enzymes, nutrients, vitamins etc. An especially suitable mode of application is, as mentioned above, the encapsulation of pharmaceuticals.

The following non-limiting examples illustrate the invention.

Example 1

For the preparation of liposomes 1,2-dimyristoyl-sn-glycero-3-phosphatidyl-sn-1′-glycerol-ammonium salt (1′-DMPG) (KSV-Chemicals Oy, Helsinki, Finland) was used as a star ting material. No impurities were detected in the lipid by thin layer chromatography on silica dioxide (Merck), using chloroform:methanol: water: ammonia at a ratio of 65:20:2:2. The lipid concentrations were determined by phosphorous analysis.

A solution of the lipid in chloroform or dichloromethane was prepared by pipetting the lipid into the solvent and then removing the solvent in a stream of nitrogen and maintaining it overnight in vacuum. Thereafter the phospholipid was suspended in 20 mM Tris/HCl, pH 7.4, 0.1 mM EDTA-buffer to a final concentration of 1 mg/ml. The hydrated lipid was treated for one hour in a Bransonic 221 type bath sonicator in a nitrogen atmosphere at 30° C. In order to remove the multilamellar liposomes the dispersion was centrifuged at a speed of 35,000 g and the supernatant containing the unilamellar liposomes was recovered and the liposomes separated.

Using the same method liposomes may prepared from the following phosphatidyl-1′-glycerols.
1,2-dioleoyl-sn-glycero-3-phosphatidyl-sn-1′-glycerol
1,2-dipalmitoyl-sn-glycero-3-phosphatidyl-sn-1′-glycerol as well as
1-palmitoyl-2-oleoyl-sn-glycero-3-phosphatidyl-sn-1′-glycerol.

In order to show the physicochemical differences of the 1′-DMPG to be used according to the invention compared to the corresponding 3′-DMPG-isomer, a liposome dispersion was prepared in a similar manner from 3′-DMPG. The lipid concentration of both liposome products was adjusted to 0.1 mg/ml. The treshhold concentrations of $Na^+$ for the formation of aggregates were measured turbidimetrically.

In order to determine the turbidity changes, a Shimadzu graphicord spectrophotometer was used having a thermostat controlled cuvette chamber. Small aliquots (35 µl) of a 3M salt solution were added. After each addition, the samples were mixed and the absorbance at 400 nM was measured three minutes after the addition of the salt. The temperature was 10° C.

In the appended Figure 1, turbidity changes are shown for liposomes containing either 1′-DMPG, 3′-DMPG or a 1 : 1-mixture thereof, as a function of sodium chloride concentration. The intersection point of $\Delta A_{400}$ with the x-axis is used as the aggregation threshhold concentration. It can be seen that the threshhold concentration for pure 1′-DMPG is about 550 mM, whereas that for a 1 : 1-mixture is somewhat lower, 500 mM, and that for pure 3′-DMPG is as low as 350 mM. It is also seen that the curve indicative of aggregation is considerably steeper for pure 3′-DMPG than for either 1′-DMPG or its 1 : 1-mixture with the 3′-isomer.

In the appended Figure 2a the aggregation of 1′ -DMPG and 3′-DMPG is shown as a function of NaCl-concentration. In Figure 2b turbidity is shown as a function of time at 10° C, indicating clearly the lesser tendency of the 1′-isomer to aggregate than the 3′-isomer. The higher affinity of the 3′-isomer for $Na^+$ is thought to be due to the slightly different favoured conformation of the head group glycerol

moiety.

The above results were confirmed by studying the salt dependant phase behaviour of the said DMPG-isomers using DSC (differential scanning calorimetry). It was thus found that the formation of the crystalline phase of 3′-DMPG occurs within a few days at 6 to 10 °C in the presence of 0.15 to 1.2 M NaCl. For a 1 : 1-mixture of the isomers the crystalline phase was found to form in a few weeks. The formation of the crystalline phase of the 1′-DMPG on the other hand, required several months to be completed.

The above studies confirm that the sodium induced aggregation of small unilamellar liposomes of 3′- and 1′-DMPG are significantly different, indicating a higher affinity of 3′-DMPG specifically for sodium ions. Also the studies indicate a superior stability of the liposome dispersions made from the 1′-isomer compared to those made from the 3′-isomer in the presence of sodium ions.

## Claims

1. A liposome containing a phosphatidyl glycerol, wherein its wall structure comprises the specific sn-3-phosphatidyl-sn-1′-glycerol isomer.

2. A lipsome according to claim 1 wherein the sn-3-phosphatidyl-sn-1′-glycerol isomer has the formula

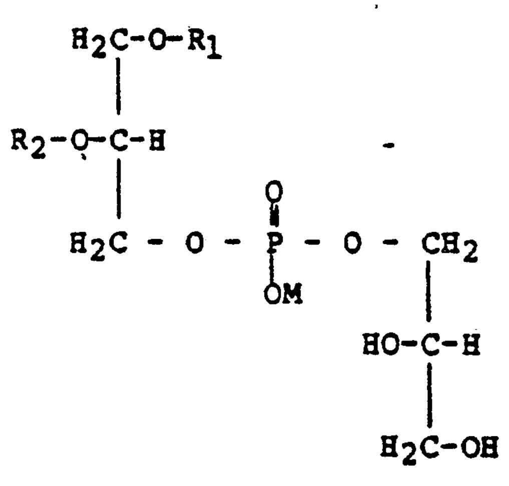

wherein $R_1$ and $R_2$ are independently straight chained alkyl-, alkenyl-, alkanoyl- or alkenoyl groups comprising 4 to 24 carbon atoms, $R_1$ and $R_2$ together containing at least 16 carbon atoms; and M is H, $NH_4^+$, $Na^+$, $K^+$, $Ca^{++}$ or $Mg^{++}$.

3. A liposome according to either of claims 1 and 2 selected from:
1,2-dimyristoyl-sn-glycero-3-phosphatidyl-sn-1′-glycerol (1′-DMPG),
1,2-dioleoyl-sn-glycero-3-phosphatidyl-sn-1′-glycerol (1′-DOPG),
1,2-dipalmitoyl-sn-glycero-3-phosphatidyl-sn-1′-glycerol (1′-DPPG),
1-palmitoyl-2-oleoyl-sn-glycero-3-phosphatidyl-sn-1′-glycerol (1′-POPG), and mixtures thereof.

4. A liposome comprising a phosphatidyl glycerol, wherein said phosphatidyl glycerol is an sn-3-phosphatidyl-sn-1′-glycerol isomer substantially free of other isomers.

5. A liposome according to any preceding claim encapsulating one or more substances selected from dyes, pharmaceuticals, enzymes, nutrients and vitamins.

6. A pharmaceutical composition comprising a liposome in accordance with any one of claims 1 to 4.

7. A process for the preparation of a liposome according to any one of claims 1 to 5, wherein a sn-3-phosphatidyl-sn1′-glycerol-isomer is dispersed in an aqueous medium and the liposomes formed are recovered.

8. A process according to claim 7 wherein the aqueous medium contains a substance to be encapsulated.

9. Use of an sn-3-phosphatidyl-sn-1′-glycerol isomer for the preparation of liposomes.

10. Use of an sn-3-phosphatidyl-sn-1′-glycerol isomer substantially free of other isomers, for the preparation of a liposome to be used in medicine.

FIG. 1

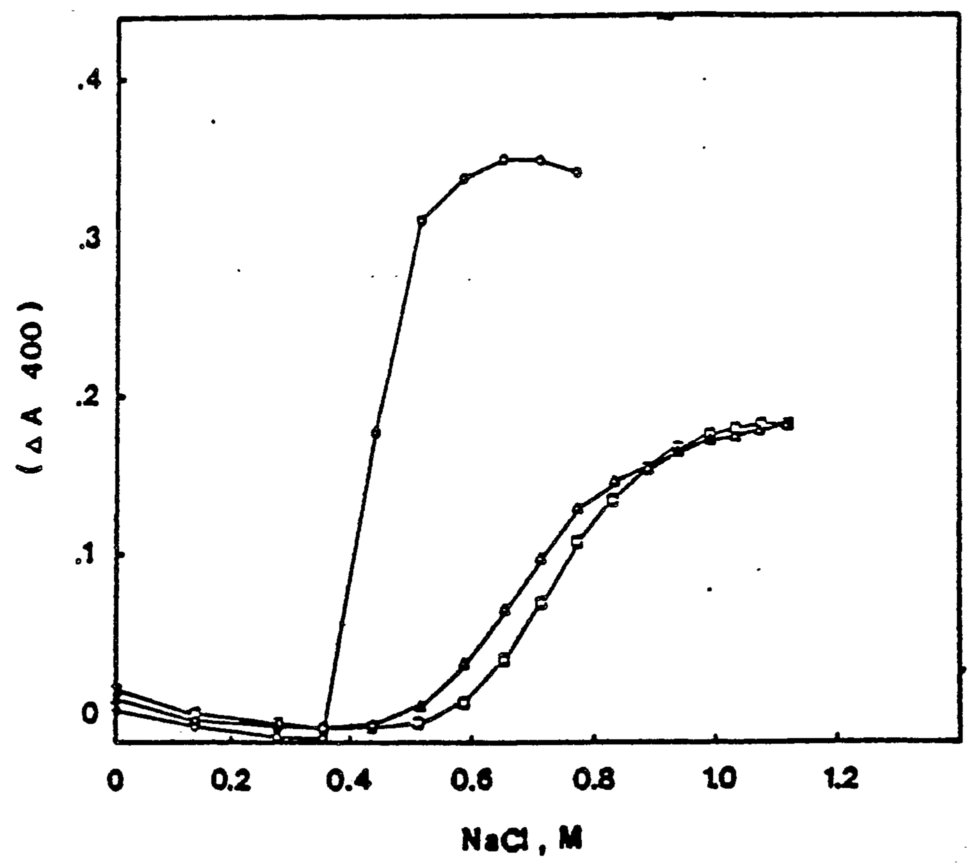


3'-DMPG (○ - ○), 1'-DMPG (□ - □), 1-1-mixture (Δ - Δ).

FIG. 2

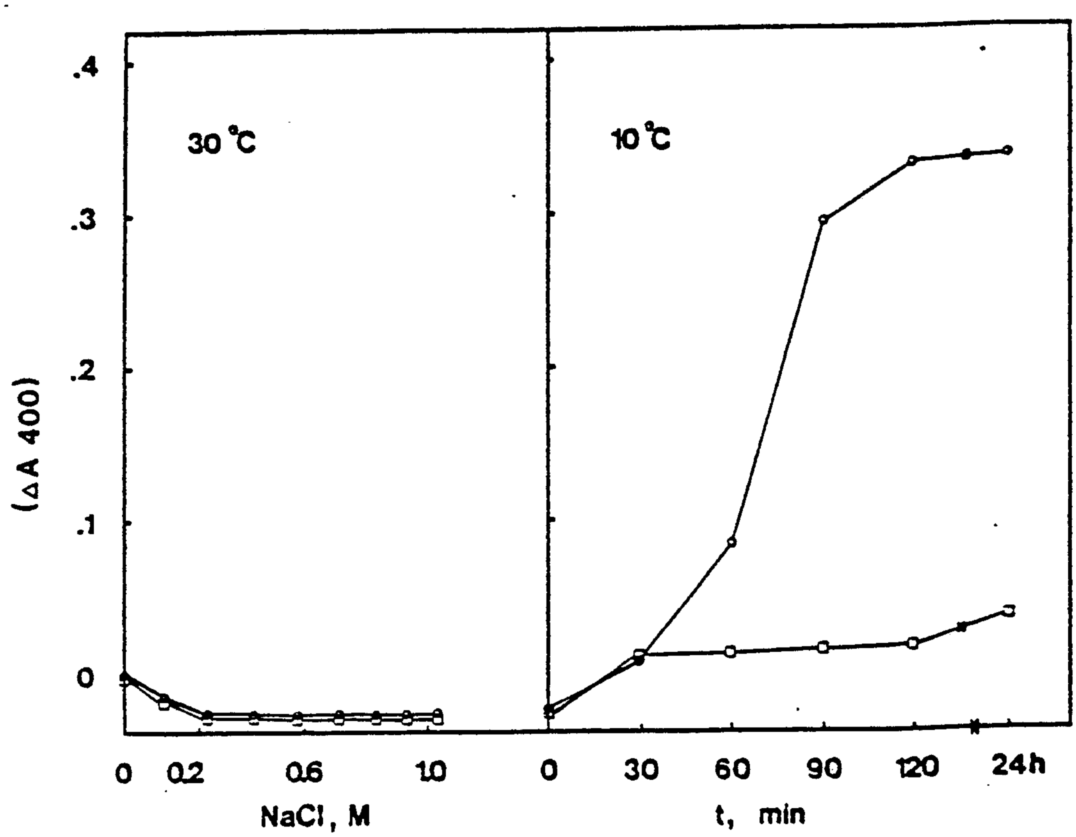

European Patent Office

# EUROPEAN SEARCH REPORT

Application Number

EP 88 31 0078

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 219 922 (VESTAR RESEARCH INC.) * Column 2, lines 21-32; claim 6 * --- | 1-10 | A 61 K 9/50 |
| A | EP-A-0 178 624 (CIBA-GEIGY AG) * Claims * ----- | 1-10 | |

TECHNICAL FIELDS SEARCHED (Int. Cl.4)

A 61 K
C 07 F

The present search report has been drawn up for all claims

| Place of search | Date of completion of the search | Examiner |
|---|---|---|
| THE HAGUE | 10-03-1989 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document